# EUROPEAN PATENT SPECIFICATION

(11) **EP 3 908 304 B1**
(45) Date of publication and mention of the grant of the patent: **10.04.2024**
(21) Application number: 20703511.4
(22) Date of filing: 07.01.2020
(51) Int. Cl.: A61K 38/04, C07K 7/08, A61P 19/00, A61P 15/10, A61P 15/08, A61P 5/50, C07K 14/415

(54) **COMPOSITIONS CONTAINING A PEPTIDE ABLE TO STIMULATE THE GPRC6A-DEPENDENT SIGNALLING PATHWAY**
ZUSAMMENSETZUNGEN MIT EINEM PEPTID ZUR STIMULATION DES GPRC6A-ABHÄNGIGEN SIGNALWEGS
COMPOSITIONS CONTENANT UN PEPTIDE APTE À STIMULER LA VOIE DE SIGNALISATION DÉPENDANTE DE GPRC6A

(30) Priority: 10.01.2019 IT 201900000367
(43) Date of publication of application: 17.11.2021
(73) Proprietor: Altergon S.A., 6900 Lugano (CH); FONDAZIONE FORESTA - ONLUS, 35128 Padova (IT)
(72) Inventor: FORESTA, Carlo, 35128 Padova (IT); DE TONI, Luca, 35128 Padova (IT); DI NISIO, Andrea, 35128 Padova (IT); DE ROCCO PONCE, Maurizio, 35128 Padova (IT); GIORI, Andrea, 6900 Lugano (CH)
(74) Representative: Bianchetti & Minoja with Trevisan & Cuonzo IPS SRL
(86) International application number: PCT/IB2020/050074
(87) International publication number: WO 2020/144561

(56) References cited:
- WO-A1-2007/093220
- WO-A2-2011/090971
- US-A1- 2012 258 194
- "Poster Presentation Abstracts", OSTEOPOROSIS INTERNATIONAL ; WITH OTHER METABOLIC BONE DISEASES, SPRINGER-VERLAG, LO, vol. 19, no. 2, 21 October 2008 (2008-10-21), pages 251-497, XP019652450, ISSN: 1433-2965, DOI: 10.1007/S00198-008-0736-6
- SONG HAIZHAO ET AL: "DietaryPhaseolus vulgarisextract alleviated diet-induced obesity, insulin resistance and hepatic steatosis and alters gut microbiota composition in mice", JOURNAL OF FUNCTIONAL FOODS, ELSEVIER BV, NL, vol. 20, 21 November 2015 (2015-11-21), pages 236-244, XP029360117, ISSN: 1756-4646, DOI: 10.1016/J.JFF.2015.10.022

## Description

The present invention relates to pharmaceutical or nutraceutical compositions comprising the peptide having the sequence NMYLPPVPPP PVVPTF in isolated form in admixture with suitable excipients. The compositions according to the invention are useful for therapeutic or adjuvant treatment of conditions that benefit from stimulation of the GPRC6A-dependent signalling pathway, i.e. male hypogonadism, osteopenia/osteoporosis, erectile dysfunction and metabolic syndrome.

### BACKGROUND OF THE INVENTION

The role of bone tissue as an endocrine organ is a subject that has aroused great scientific interest in the last ten years. Initial studies of animal models suggested that osteocalcin (OCN), a small protein of osteoblastic origin which is normally involved in mineralisation of extracellular bone matrix in its γ-carboxylated form, may exhibit systemic effects after decarboxylation. In particular, uncarboxylated osteocalcin (ucOCN) appeared to be a direct stimulator of insulin release from the pancreatic islets, a protective factor in the maintenance of insulin sensitivity in the main target organs of the hormone, such as adipose tissue and muscle, and a regulator of bone mass and male fertility through control of the production and release of testosterone (T) by the Leydig cells in the testicles [Lee Cell. 2007; Ferron Proc Natl Acad Sci USA. 2008; Pi PLoS One. 2008; Rached J Clin Invest. 2010; Ferron Cell. 2010; Oury Cell. 2011]. *In vitro* and *in vivo* studies identified the GPRC6A receptor as the mediator of the systemic activity of ucOCN [Pi J Biol Chem. 2005; Pi J Bone Miner Res. 2011; Wei Diabetes. 2014]. Subsequent studies clarified that the endocrine role of ucOCN through the GPRC6A receptor was also preserved in humans, confirming the role of this novel multi-organ endocrine axis [Foresta J Clin Endocrinol Metab. 2010; Oury. J Clin Invest. 2013; De Toni Endocrinology. 2014; De Toni J Clin Endocrinol Metab. 2016; Di Nisio Clin Endocrinol (Oxf). 2017; Parker J Bone Miner Res. 2018].

The crucial characteristic of GPRC6A is that it is a metabotropic receptor, i.e. can be activated by a range of small molecules of metabolic interest such as basic amino acids (e.g. arginine and ornithine) and metal cations (e.g. calcium and magnesium) [Pi J Biol Chem. 2005]. *In vitro* studies conducted in the early 2010s suggested that GPRC6A could represent the T membrane receptor, i.e. the mediator of the "non-genomic" or rapid effects of androgens [Pi J Biol Chem. 2010; Pi Mol Endocrinol. 2015]. The correctness of this hypothesis was subsequently verified in numerous *in vitro* studies of cell lines and primary cell cultures characterised by constitutive expression of GPRC6A [Ko. J Biol Chem. 2014; Pi Mol Endocrinol. 2015; O'Hara FASEB J. 2015].

According to some authors, the hypothesis that GPRC6A represents the common molecular target of some highly chemically different entities such as amino acids, metal cations and steroids constitutes a biological model with some highly critical aspects [Clemmensen Br J Pharmacol. 2014]. In this respect, it should be borne in mind that the majority of T is present in the peripheral circulation as a complex with a serum globulin known as "sex-hormone-binding globulin" (SHBG) [Mean Clin Chim Acta. 1977]. Moreover, numerous studies have demonstrated a significant association between low plasma levels of SHBG and clinical signs of metabolic alterations such as increased insulin resistance and increased risk of type 2 diabetes [Birkeland J Clin Endocrinol Metab. 1993; Tsai Diabetes Care. 2004; Muller J Clin Endocrinol Metab. 2005; Pitteloud Diabetes Care. 2005; Ding N Engl J Med. 2009]. These factors, together with the fact that both ucOCN and SHBG are proteins, suggested that SHBG may act as a mediator in the interaction between T and GPRC6A. That hypothesis was confirmed in a recent study wherein, using an integrated computational-experimental approach, a common bonding site for interaction with both ucOCN and SHBG was identified in the extracellular domain of GPRC6A [De Toni Endocrinology 2016]. WO2009073544 proposes the possible therapeutic use of agents able to regulate the level and/or activity of GPRC6A, especially for the treatment of obesity and diabetes. WO2011090971 discloses peptides snd methods for treating male reproductive disorders. WO 2007093220 discloses peptides snd methods for treating osteoporosis.

### Description of the invention

It has now been discovered that an amino-acid sequence exhibits close homology (62.5% amino-acid identity; 75% structural similarity) with domain 141-161 of SHBG (SHBG₁₄₁₋₁₆₁), corresponding to a protein loop with high flexibility responsible for the interaction between ucOCN and GPRC6A.

It has also been found that said peptide sequence (NMYLPPVPPPPWPTF, SEQ ID 1, P80762) stimulates insulin and testosterone release in experimental models of cell line cultures.

The object of the invention is therefore pharmaceutical or nutraceutical compositions comprising the peptide having the sequence NMYLPPVPPP PVVPTF in isolated form in admixture with suitable excipients.

The compositions according to the invention are useful in the treatment of conditions that benefit from stimulation of the GPRC6A-dependent signalling pathway, i.e. male hypogonadism, osteopenia/osteoporosis, erectile dysfunction and metabolic syndrome.

The peptide NMYLPPVPPPPVVPTF can be prepared by conventional peptide synthesis methods.

The compositions according to the invention can be administered orally, transdermally or parenterally.

Examples of suitable forms of administration include capsules, tablets, solutions, suspensions, gels, transdermal patches and the like. The peptide can be combined with other ingredients having complementary or otherwise useful activity such as vitamins, amino acids and antioxidants, minerals such as zinc and magnesium salts, and phytotherapeutic extracts (such as *Panax ginseng, Ginkgo biloba,* green tea, *Vitis vinifera, Vaccinium myrtillus, Serenoa repens, Aloe vera, Cynara scolymus, Citrus aurantium* and *Zingiber officinalis).*

The effective doses of the peptides will be determined by the skilled person on the basis of pre-clinical and clinical tests. The doses will depend on various factors, such as the type and severity of the patient's disorder or condition, and the patient's weight, sex and age. However, an average daily dose could range from 10 to 1000 mg of peptide, or the equivalent of the extract containing it.

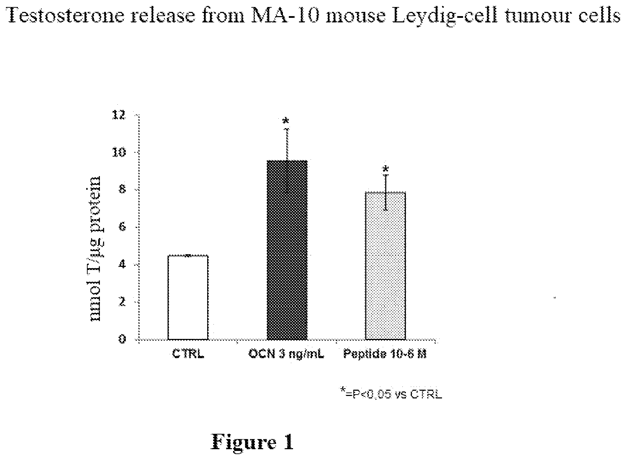

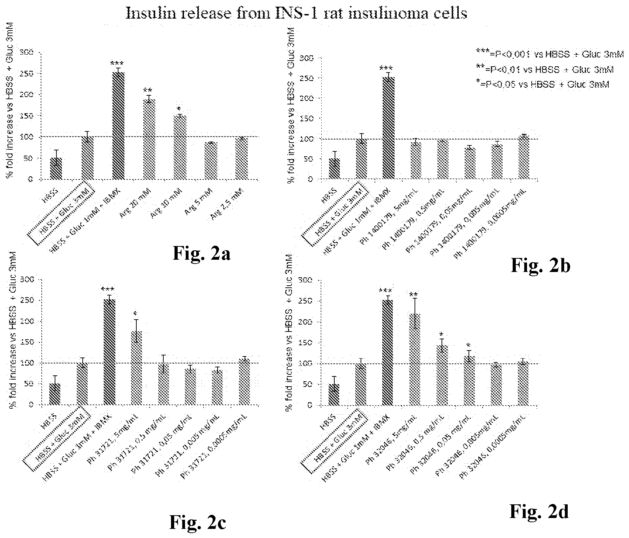

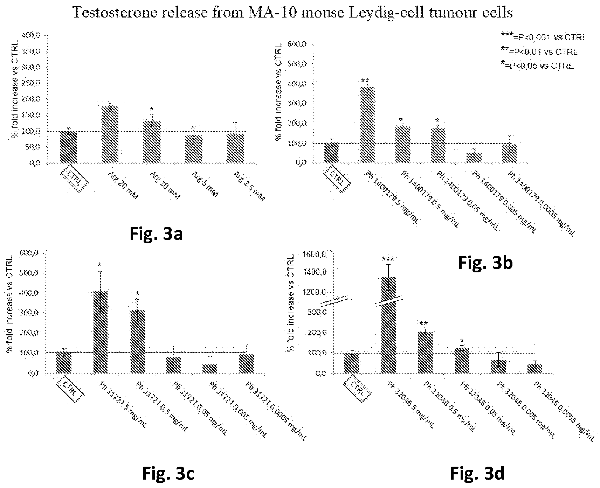

The efficacy of the formulations according to the invention can be deduced from *in vitro* studies conducted on INS-1 rat insulinoma and MA-10 mouse Leydig cell tumour cell lines, using kidney bean flour of commercial origin. The results, reported in the experimental section below, demonstrated the release of insulin (Fig. 2) and testosterone (Figs. 1 and 3). Expression of GPRC6A was confirmed in both cell lines [Brar Diabetes. 2017; De Toni Endocrinology. 2014].

The following examples illustrate the invention in greater detail.

### Example 1 Testosterone release from MA-10 mouse Leydig-cell tumour cell cultures

MA-10 mouse Leydig-cell tumour cells, cultured to 90% confluence in 24-well multiwell plates, were stimulated with different agonists for 12 hours at 37°C under sterile conditions, according to an already validated protocol [Cormier Cell Biol Toxicol. 2018]. The control (CTRL) contained no agonists. The results relate to testosterone release, expressed as ng of testosterone (T) per µg of total protein, obtained from cell extract. The histograms show the effect on testosterone release of uncarboxylated osteocalcin (OCN, 3 ng/mL) and peptide SEQ ID 1 (Peptide, 10⁻⁶M). Significance: *=P<0.05 vs. CTRL.

### Example 2 Insulin release from INS-1 murine insulinoma cell cultures

INS-1 rat insulinoma cells, cultured to 90% confluence in 24-well multiwell plates, were stimulated with different combinations of secretagogue agonists for 2 hours at 37°C under sterile conditions, according to an already validated protocol [Hohmeier Diabetes. 2000]. The results relate to insulin release, expressed as the normalised variation in low-concentration glucose (Gluc 3mM) at the baseline control. Stimulation with the phosphodiesterase inhibitor IBMX (Glue 1mM + IBMX) represents the maximum secretagogue stimulation and positive control of the experiment. The four histograms in Figure 2a show the effect on insulin release of arginine (Arg, at concentrations ranging from 2.5 to 20 mM), a known GPRC6A agonist, while the release of three Phaseolus extracts (Ph1400179, Ph31721 and Ph32046, at concentrations ranging from 0.0005 to 5 mg/mL), is shown in figures 2b-d respectively. Significance: *, ** and ***=P<0.05, 0.01 and 0.001 respectively vs the baseline control.

### Example 3 Testosterone release from MA-10 mouse Leydig-cell tumour cell cultures

MA-10 mouse Leydig-cell tumour cells, cultured to 90% confluence in 24-well multiwell plates, were stimulated with different agonists for 12 hours at 37°C under sterile conditions, according to an already validated protocol [Cormier Cell Biol Toxicol. 2018]. The control (CTRL) contained no agonists The results relate to testosterone release, expressed as normalised variation vs the CTRL. The four histograms in Figure 3a show the effect on testosterone release of arginine (Arg, at concentrations ranging from 2.5 to 20 mM), a known GPRC6A agonist, while the release of three Phaseolus extracts (Ph1400179, Ph31721 and Ph32046, at concentrations ranging from 0.0005 to 5 mg/mL), is shown in figures 3b-d respectively. Significance: *, ** and ***=P<0.05, 0.01 and 0.001 respectively vs the baseline control.

### REFERENCES

Birkeland KI, Hanssen KF, Torjesen PA, Vaaler S. Level of sex hormone binding globulin is positively correlated with insulin sensitivity in men with type 2 diabetes. J Clin Endocrinol Metab. 1993; 76(2):275-278.

Brar GS, Barrow BM, Watson M, Griesbach R, Choung E, Welch A, Ruzsicska B, Raleigh DP, Zraika S. Neprilysin Is Required for Angiotensin-(1-7)'s Ability to Enhance Insulin Secretion via Its Proteolytic Activity to Generate Angiotensin-(1-2). Diabetes. 2017 Aug;66(8):2201-2212.

Clemmensen C, Smajilovic S, Wellendorph P, Bräuner-Osborne H. The GPCR, class C, group 6, subtype A (GPRC6A) receptor: from cloning to physiological function. Br J Pharmacol. 2014 Mar;171(5):1129-41. doi: 10.1111/bph. 12365.

Cormier M, Ghouili F, Roumaud P, Bauer W, Touaibia M, Martin LJ. Influences of flavones on cell viability and cAMP-dependent steroidogenic gene regulation in MA-10 Leydig cells. Cell Biol Toxicol. 2018 Feb;34(1):23-38.

De Toni L, De Filippis V, Tescari S, Ferigo M, Ferlin A, Scattolini V, Avogaro A, Vettor R, Foresta C. Uncarboxylated osteocalcin stimulates 25-hydroxy vitamin D production in Leydig cell line through a GPRC6a-dependent pathway. Endocrinology. 2014 Nov;155(11):4266-74.

De Toni L, Di Nisio A, Speltra E, Rocca MS, Ghezzi M, Zuccarello D, Turiaco N, Ferlin A, Foresta C. Polymorphism rs2274911 of GPRC6A as a Novel Risk Factor for Testis Failure. J Clin Endocrinol Metab. 2016 Mar;101(3):953-61.

De Toni L, Guidolin D, De Filippis V, Tescari S, Strapazzon G, Santa Rocca M, Ferlin A, Plebani M, Foresta C. Osteocalcin and Sex Hormone Binding Globulin Compete on a Specific Binding Site of GPRC6A. Endocrinology. 2016 Nov; 157(11):4473-4486.

Di Nisio A, Rocca MS, Fadini GP, De Toni L, Marcuzzo G, Marescotti MC, Sanna M, Plebani M, Vettor R, Avogaro A, Foresta C. The rs2274911 polymorphism in GPRC6A gene is associated with insulin resistance in normal weight and obese subjects. Clin Endocrinol (Oxf). 2017 Feb;86(2):185-191.

Ding EL, Song Y, Manson JE, Hunter DJ, Lee CC, Rifai N, Buring JE, Gaziano JM, Liu S. Sex hormone-binding globulin and risk of type 2 diabetes in women and men. N Engl J Med. 2009 Sep 17;361(12): 1152-63.

Ferron M, Hinoi E, Karsenty G, Ducy P. Osteocalcin differentially regulates beta cell and adipocyte gene expression and affects the development of metabolic diseases in wild-type mice. Proc Natl Acad Sci USA. 2008 Apr 1;105(13):5266-70.

Ferron M, Wei J, Yoshizawa T, Del Fattore A, DePinho RA, Teti A, Ducy P, Karsenty G. Insulin signaling in osteoblasts integrates bone remodeling and energy metabolism. Cell. 2010 Jul 23;142(2):296-308.

Foresta C, Strapazzon G, De Toni L, Gianesello L, Calcagno A, Pilon C, Plebani M, Vettor R. Evidence for osteocalcin production by adipose tissue and its role in human metabolism. J Clin Endocrinol Metab. 2010 Jul;95(7):3502-6.

Hohmeier HE, Mulder H, Chen G, Henkel-Rieger R, Prentki M, Newgard CB. Isolation of INS-1-derived cell lines with robust ATP-sensitive K+ channel-dependent and -independent glucose-stimulated insulin secretion. Diabetes. 2000 Mar;49(3):424-30.

Ko E, Choi H, Kim B, Kim M, Park KN, Bae IH, Sung YK, Lee TR, Shin DW, Bae YS. Testosterone stimulates Duox1 activity through GPRC6A in skin keratinocytes. J Biol Chem. 2014 Oct 17;289(42):28835-45.

Lee NK, Sowa H, Hinoi E, Ferron M, Ahn JD, Confavreux C, Dacquin R, Mee PJ, McKee MD, Jung DY, Zhang Z, Kim JK, Mauvais-Jarvis F, Ducy P, Karsenty G. Endocrine regulation of energy metabolism by the skeleton. Cell. 2007 Aug 10;130(3):456-69.

Mean F, Pellaton M, Magrini G. Study on the binding of dihydrotestosterone, testosterone and oestradiol with sex hormone binding globulin. Clin Chim Acta. 1977;80(1):171-180.

Muller M, Grobbee DE, den Tonkelaar I, Lamberts SW, van der Schouw YT. Endogenous sex hormones and metabolic syndrome in aging men. J Clin Endocrinol Metab. 2005;90(5):2618-2623.

O'Hara L, McInnes K, Simitsidellis I, Morgan S, Atanassova N, Slowikowska-Hilczer J, Kula K, Szarras-Czapnik M, Milne L, Mitchell RT, Smith LB. Autocrine androgen action is essential for Leydig cell maturation and function, and protects against late-onset Leydig cell apoptosis in both mice and men. FASEB J. 2015 Mar;29(3):894-910.

Oury F, Ferron M, Huizhen W, Confavreux C, Xu L, Lacombe J, Srinivas P, Chamouni A, Lugani F, Lejeune H, Kumar TR, Plotton I, Karsenty G. Osteocalcin regulates murine and human fertility through a pancreas-bone-testis axis. J Clin Invest. 2013 Jun;123(6):2421-33.

Oury F, Sumara G, Sumara O, Ferron M, Chang H, Smith CE, Hermo L, Suarez S, Roth BL, Ducy P, Karsenty G. Endocrine regulation of male fertility by the skeleton. Cell. 2011 Mar 4;144(5):796-809.

Parker L, Lin X, Garnham A, McConell G, Stepto NK, Hare DL, Byrnes E, Ebeling PR, Seeman E, Brennan-Speranza TC, Levinger I. Glucocorticoid-Induced Insulin Resistance in Men Is Associated With Suppressed Undercarboxylated Osteocalcin. J Bone Miner Res. 2018 Aug 23. doi: 10.1002/jbmr.3574.

Pi M, Chen L, Huang MZ, Zhu W, Ringhofer B, Luo J, Christenson L, Li B, Zhang J, Jackson PD, Faber P, Brunden KR, Harrington JJ, Quarles LD. GPRC6A null mice exhibit osteopenia, feminization and metabolic syndrome. PLoS One. 2008;3(12):e3858.

Pi M, Faber P, Ekema G, Jackson PD, Ting A, Wang N, Fontilla-Poole M, Mays RW, Brunden KR, Harrington JJ, Quarles LD. Identification of a novel extracellular cation-sensing G-protein-coupled receptor. J Biol Chem. 2005 Dec 2;280(48):40201-9.

Pi M, Kapoor K, Wu Y, Ye R, Senogles SE, Nishimoto SK, Hwang DJ, Miller DD, Narayanan R, Smith JC, Baudry J, Quarles LD. Structural and Functional Evidence for Testosterone Activation of GPRC6A in Peripheral Tissues. Mol Endocrinol. 2015 Dec;29(12):1759-73.

Pi M, Parrill AL, Quarles LD. GPRC6A mediates the non-genomic effects of steroids. J Biol Chem. 2010 Dec 17;285(51):39953-64.

Pi M, Wu Y, Quarles LD. GPRC6A mediates responses to osteocalcin in β-cells in vitro and pancreas in vivo. J Bone Miner Res. 2011 Jul;26(7): 1680-3.

Pitteloud N, Mootha VK, Dwyer AA, et al. Relationship between testosterone levels, insulin sensitivity, and mitochondrial function in men. Diabetes Care. 2005;28(7):1636-1642.

Rached MT, Kode A, Silva BC, Jung DY, Gray S, Ong H, Paik JH, DePinho RA, Kim JK, Karsenty G, Kousteni S. FoxO1 expression in osteoblasts regulates glucose homeostasis through regulation of osteocalcin in mice. J Clin Invest. 2010 Jan;120(1):357-68.

Tsai EC, Matsumoto AM, Fujimoto WY, Boyko EJ. Association of bioavailable, free, and total testosterone with insulin resistance: influence of sex hormone-binding globulin and body fat. Diabetes Care. 2004;27(4):861-868.

Wei J, Hanna T, Suda N, Karsenty G, Ducy P. Osteocalcin promotes β-cell proliferation during development and adulthood through Gprc6a. Diabetes. 2014 Mar;63(3):1021-31.

## Claims

1. Pharmaceutical or nutraceutical compositions comprising the peptide having the sequence NMYLPPVPPP PVVPTF in isolated form.

2. Compositions according to claim 1 further comprising other active ingredients having complementary or synergic activity.

3. Compositions according to claims 1-2 for use in the treatment of male hypogonadism, osteopenia/osteoporosis, erectile dysfunction and metabolic syndrome.

## Patentansprüche

1. Pharmazeutische oder nutrazeutische Zusammensetzungen, die das Peptid mit der Sequenz NMYLPPVPPP PVVPTF in isolierter Form umfassen.

2. Zusammensetzungen nach Anspruch 1, die ferner andere aktive Bestandteile mit komplementärer oder synergistischer Wirkung umfassen.

3. Zusammensetzungen nach den Ansprüchen 1 bis 2 zur Verwendung bei der Behandlung von männlichem Hypogonadismus, Osteopenie/Osteoporose, erektiler Dysfunktion und metabolischem Syndrom.

## Revendications

1. Compositions pharmaceutiques ou nutraceutiques comprenant le peptide ayant la séquence NMYLPPVPPP PVVPTF sous forme isolée.

2. Compositions selon la revendication 1, comprenant en outre d'autres ingrédients actifs ayant une activité complémentaire ou synergique.

3. Compositions selon l'une des revendications 1-2 pour l'utilisation dans le traitement de l'hypogonadisme masculin, de l'ostéopénie/ostéoporose, du dysfonctionnement érectile et du syndrome métabolique.
